# EUROPEAN PATENT SPECIFICATION

(11) **EP 2 240 427 B1**
(45) Date of publication and mention of the grant of the patent: **11.02.2015**
(21) Application number: 08865580.8
(22) Date of filing: 22.12.2008
(51) Int. Cl.: B01D 53/40, B01D 53/78, C07C 37/08

(54) **A process for oxidizing cumene to cumene hydroperoxide**
Verfahren zur Oxidation von Cumol zu Cumolhydroperoxid
Procédé d'oxydation du cumène à l'hydroperoxyde de cumène

(30) Priority: 20.12.2007 EP 07150212
(43) Date of publication of application: 20.10.2010
(73) Proprietor: Borealis Technology Oy, 06101 Porvoo (FI)
(72) Inventor: PENTTI, Ismo, A-1210 Wien (AT); SAUKONOJA, Jouni, FIN-06400 Porvoo (FI); PAKALA, Timo, FIN-06400 Porvoo (FI); MANNERLA, Anja, FIN-02730 Espoo (FI)
(74) Representative: Golding, Louise Ann
(86) International application number: PCT/EP2008/011008
(87) International publication number: WO 2009/080341

(56) References cited:
- US-A- 5 144 807
- US-A- 5 220 103
- US-A1- 2002 011 150
- US-A1- 2005 126 393

## Description

The present invention concerns a process for removing impurities from air for use in the oxidation of cumene.

Phenol production is generally based on the oxidation of cumene to cumene hydroperoxide (CHP) using air as a source of oxygen. For achieving an efficient production process, the purity of the raw materials is essential.

Small quantities of impurities may get into the cumene oxidation phase with the cumene feed. Other impurities may get into the oxidation phase with the air. Some impurity components react with the oxygen in the air and thus increase the number of unwanted side reactions, thereby lowering the efficiency of the main reaction. Components that slow down cumene oxidation to CHP are for example phenol, α-methyl styrene (AMS) and salts.

Some components, on the other hand, may cause undesired premature cleavage of the CHP, i.e. cleavage occurring before the actual (i.e. desired) cleavage step. Among these are acidic components, such as the mentioned phenol and formaldehyde.

A high AMS concentration causes a decrease of the pH in oxidation and therefore early cleavage of CHP into phenol and acetone. Phenol, in addition to the early cleavage, slows down or inhibits the cumene oxidation process even in low concentrations.

An extremely harmful impurity in the cumene oxidation phase is sulfur, which stops the formation of CHP. In the reaction mechanism sulfur has two effects:
- sulfur reacts with free radicals, thereby preventing the formation of new radicals
- sulfur gets oxidized into sulfur oxides and from thereon into sulfuric acid, which causes premature CHP decomposition into phenol and acetone.

In air, the sulfur is commonly present as sulfur oxides, e.g. SO₂. Organic sulfur solutions, such as thiophene, have the same effect on CHP formation as sulfur.

A further impurity, which is present in air and is harmful for the oxidation process, is carbon dioxide (CO₂). Carbonates, originating for example from CO₂, increase fouling during the oxidation phase and in the subsequent concentration (i.e. purification) of CHP. Alkali components also cause slow CHP decomposition into acetophenone and carbinol, thus causing cumene losses. Carry-over of alkali components to the cleavage phase of the phenol production process may neutralize the acid used to catalyze the cleavage reaction.

Thus, when using air from industrialised areas, it is important that this is scrubbed before use to reduce (preferably to remove) any traces of sulfur dioxide, carbon dioxide or other acidic gases that may pass with the air into oxidation columns, such as those which may be used in the production of cumene. As mentioned above, sulfur dioxide converts into sulfuric acid on oxidation, which causes premature CHP decomposition into phenol and acetone. Phenol in small quantities slows down, and in larger quantities, inhibits the formation of CHP.

Many of the mentioned impurities can be removed by a caustic wash, as performed for a cumene feed in US 5,120,902 and US 5,220,103. Amongst other things, a caustic wash has the benefit of neutralizing any acids, which may slow down the oxidation reaction or cause CHP decomposition.

However, the by-products of caustic treatment of air may also be harmful, particularly when this is used in a dry oxidation procedure. For example, any remaining salts can slowly block the spargers (air inlets) and the air distributors of the oxidation reactors, whereby these reactors require periodic cleaning (purification).

The present invention provides a process for the treatment of air which is capable of reducing (e.g. removing) harmful substances from air to be used in an oxidation process in the production of phenol from cumene.

Particularly, the present invention provides a process for removing substances from air, which substances could slow down or prevent the formation of CHP from cumene and/or could cause blocking of air inlets or outlets when using the air as a source of oxygen in a process for the oxidation of cumene to CHP.

The present invention concerns a process for reducing (e.g. removing) impurities in air for use in the oxidation of cumene, which process comprises providing an air feed which comprises (e.g. consists essentially of) air and one or more acidic components, and contacting the air with an aqueous caustic solution which comprises at least one alkaline compound whereby to neutralize at least one of said acidic components in the air. Preferably, inorganic salts are formed as a result of carrying out this particular step of the process. Following separation of the air stream from the caustic solution, the treated air is contacted with water whereby to dissolve any residual caustic solution and/or salts. Separation of the washed air stream from the water provides an air stream which is particularly suitable for use in the oxidation of cumene, e.g. in the dry oxidation of cumene.

Considerable advantages are obtained by means of the invention. Thus, the present invention provides an efficient purification process that reduces (e.g. removes) harmful substances and trace elements that commonly are present in industrial air. Using the process of the invention extends the period of time between the required cleaning (purification) of the oxidation reactors.

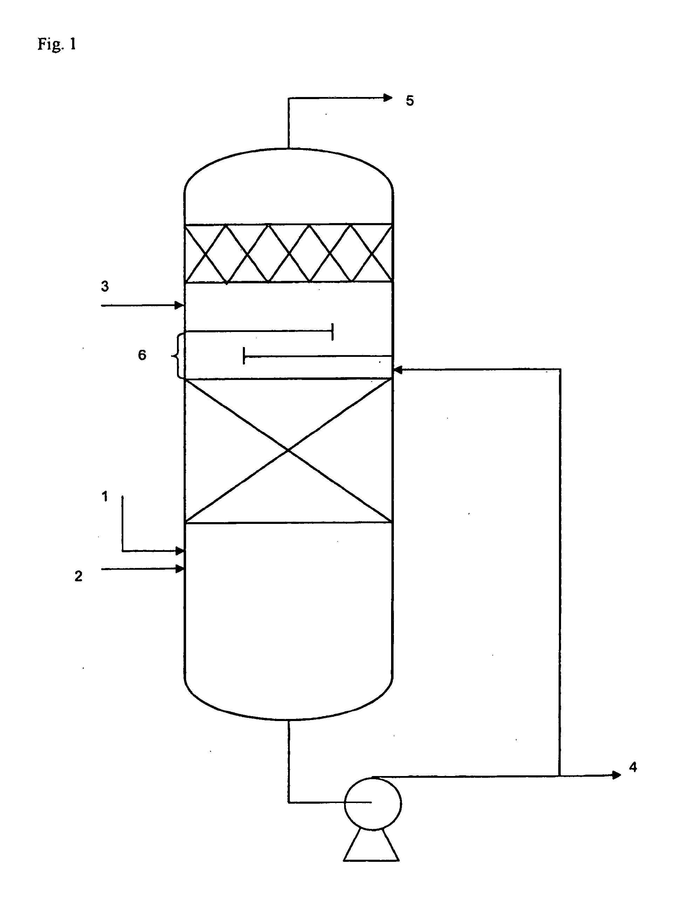

Next, the invention will be described more closely with reference to the attached Figure 1 which is a schematic drawing of a preferred embodiment of an apparatus suitable for performing the process of the present invention.

The present invention concerns a process for reducing (e.g. removing) impurities in air to be used in the oxidation of cumene, which process comprises providing an air feed comprising (e.g. consisting essentially of) air and one or more acidic components, and contacting the air with an aqueous caustic solution which contains at least one alkaline compound, to neutralize the acidic components in the air, e.g. to form inorganic salts.

Particularly, the present invention concerns a purification process comprising separating a first purified air stream from the caustic solution. This may conveniently be achieved by passing the air through one or more, preferably at least two, column trays. The first purified air stream is then contacted with water (preferably fresh water) to dissolve any residual caustic solution and/or salts in the water. Thereafter, a second purified air stream is separated from the water containing residual caustic solution and/or salts.

The purification of the air can be achieved, for example, using a purification system consisting of a purification column, which comprises the following parts (Fig. 1):
- 1: an air inlet
- 2: a caustic solution inlet
- 3: a water inlet
- 4: an outlet for the mixture of caustic solution and water
- 5: an air outlet
- 6: column trays.

Thus, the purification column comprises an air inlet 1 for conducting air into the column and a caustic solution inlet 2 for conducting a caustic solution into the column (both positioned in the lower part of the column), a water inlet 3 for conducting fresh water into the column (positioned in the upper part of the column), an outlet 4 for the mixture of caustic solution and water used for purification (positioned in the pump discharge line for conducting the caustic solution and the water out from the column), an air outlet 5 (positioned in the upper part of the column) for conducting air out from the column, and at least two column trays 6 (positioned between the caustic solution inlet 2 and the water inlet 3). Column trays 6 allow for the separation of the caustic solution from the air before the air contacts the water conducted into the column from the water inlet 3.

The purification process of the present invention may generally be used to purify industrial air. Preferably, the process and the column are used as part of a phenol production process, wherein phenol and acetone are produced through the oxidation of cumene to cumene hydroperoxide (CHP) and the subsequent steps of concentrating the CHP, cleaving the CHP into phenol, acetone and other cleavage products, washing and desalting the cleavage products and finally separating the acetone from the phenol and purifying both desired products.

When used as part of a phenol production process, preferably the CHP formed during the oxidation is further processed, e.g. by increasing its concentration in a series of concentration steps, and subjecting it to a cleavage process. According to this preferred aspect, the obtained cleavage product mixture is conducted further to a distillation section, wherein the cleavage product mixture (which may contain, for example, phenol, acetone, water, cumene, AMS, hydroxyacetone, mesityl oxide, acetophenone, carbinol, mesityl oxide and heavy hydrocarbons) is distilled.

Particularly preferably, the treated air is used in a dry oxidation process, such as that which may be used in the oxidation of cumene. By "dry oxidation" it is intended that all components used in the oxidation of cumene (i.e. cumene and air) should be substantially free from water droplets (i.e. free water) and that no additional process water is added during the oxidation procedure, i.e. no water-emulsion is produced. Where any water is present during a dry oxidation process, this will generally be at a level below saturation point. A particular feature of a dry oxidation process is the absence of any purification stage between cumene oxidation and concentration of CHP.

Air, as used in the present invention, for example in connection with the air feed, encompasses industrial air, air enriched with oxygen and concentrated oxygen. By "industrial air" is meant air of the quality which may be present in an industrialised area and which typically will contain undesirable impurities such as acidic gases. Concentrated oxygen is any gaseous mixture containing up to 100% oxygen, preferably about 22-80% oxygen, the rest preferably being mostly inert gases. Air suitable for use in the invention will generally be free from volatile hydrocarbons.

The water for use in the process of the invention may be fresh water or recycled water, however, this is preferably fresh water. Preferably, the water (e.g. fresh water) used in the process of the invention is essentially free from acidic and alkaline components. Where any acidic or alkaline components are present, typically these will each be present in an amount of less than 10 ppm, e.g. less than 0.1 ppm. Particularly preferably, the water is essentially free from chlorides, particularly having a chloride concentration of less than 10 ppm, e.g. less than 0.1 ppm. More preferably, the water is condensed water.

Generally, the air to be used in any subsequent oxidation process is maintained at atmospheric pressure and is cooled down to a temperature of 20-80°C, preferably about 30-60°C. The temperature of the air has an effect on the amount of evaporated water being conducted to the step of oxidation, since a high air temperature, particularly a temperature that rises to 100°C or above, causes evaporation of water. Alternatively, the air may have a slight over-pressure, i.e., a pressure above the pressure of the subsequent process steps, for example any oxidation step such as may be used in the production of phenol from cumene.

After any optional pressurization and cooling, the air is conducted through an air inlet 1 to the bottom part of the purification column, where it is also possible to feed fresh caustic. The column preferably has at least two column trays, and above the upper one clean water is fed. The air is scrubbed in the column by circulating water and a caustic solution in the column and in a circulation line. With the circulation flow, the scrub solution of the column is recycled and the need for fresh caustic is reduced. For example, the mixture of caustic solution and water may be recycled to the caustic solution inlet.

The aqueous caustic solution preferably contains from about 0.1 to about 5% by weight, more preferably from about 0.3 to 1.0% by weight, caustic and the preferred caustic solution is sodium hydroxide (NaOH). A 0.1-5% sodium hydroxide (NaOH) solution is particularly preferred.

The caustic solution may be fresh caustic or, alternatively, this may be recycled. A mixture of fresh and recycled caustic solutions may also be used.

According to a preferred embodiment of the present invention, the fresh caustic is fed into the column through a separate fresh caustic inlet positioned between the caustic inlet 2 and the air inlet 1 (not shown in Fig. 1). Alternatively, the fresh caustic may be fed into the circulation line through a fresh caustic inlet and subsequently together with the circulation flow through the caustic solution inlet 2 into the column.

The amount of fresh water introduced into the column is generally constant. The amount of caustic (e.g. fresh caustic) fed into the circulation is generally limited to a level that keeps the concentration of caustic (e.g. NaOH) in the circulation at a level of 0.1-5%.

A portion of the aqueous solution is constantly removed from the column and the circulation in order to maintain a constant caustic (e.g. NaOH) concentration and to maintain constant liquid levels in the column. This portion of aqueous solution may for example be recycled to a cumene washing step or to a step of washing off gases in a phenol production process.

According to an embodiment of the present invention, alkali liquid drops are prevented from following the air to the oxidation reactors by first passing the air through one or more, preferably at least two, column trays 6 and by adding water (e.g. pure water) to a level that is higher up in the column than the column trays.

According to a preferred aspect of the invention, pure water is sprayed into the column as a mist.

A demister is preferably positioned in the upper half of the column, which demister is a structure or device that catches liquid drops and droplets from the air before the air is fed to the oxidation reactors. The demister preferably removes substantially all free water droplets from the air (which may contain any residual caustic solution and/or salts). The air feed which is produced is particularly suitable for use in a dry oxidation procedure, such as that employed in the oxidation of cumene.

The column preferably contains both column trays 6 and a demister. Although the demister serves to remove residual caustic from the air, this alone is not sufficiently efficient in all circumstances. The column trays and the water wash are still advantageous for removing any traces of the caustic solution from the air.

The column trays may be for example in the shape of so-called bubble-cap or valve-cap trays. These function by achieving good contact between the liquid on the tray and the gas, such as the air, with the help of bubble-caps or valve caps located at each perforation on a perforated tray. These caps form a weir that prevents the liquid from flowing through the perforations and, thus, promote the formation of vapor bubbles that may flow through the thin layer of liquid maintained on each tray.

After implementation of the purification process of the present invention, the air being fed to the oxidation reactors typically contains less than 350 ppm of acidic by-products, such as less than 0.1 ppm of sulfur dioxide and less than 300 ppm of carbon dioxide.

The following non-limiting Example is intended to further illustrate the invention:

### Example

With reference to Fig. 1, fresh air (5 bars, 40°C) is introduced into the lower portion of a purification column at a flow rate of 16 tonnes per hour. The air initially contains acidic components (e.g. CO₂ and SO₂).

A 5% aqueous NaOH solution is circulated at a flow rate of 10.5 tonnes per hour into contact with the air mixture. The air/vapour mixture is passed through two column trays positioned in the upper portion of the column whereby to separate the NaOH solution from the air stream.

A fine mist of fresh water (water condensate) at ambient temperature is fed into the upper portion of the column at a rate of 400 kg per hour. On contact with the air stream this dissolves residual caustic solution and salts present in the air stream.

A demister removes any residual free water droplets from the air before this is fed to a cumene oxidation reactor.

## Claims

1. A process for oxidising cumene to cumene hydroperoxide, which process comprises:
- providing an air feed which comprises air and one or more acidic components,
- contacting the air feed with an aqueous caustic solution, which contains at least one alkaline compound to neutralize at least one of said acidic components in the air feed, whereby salts are formed,
- separating a first purified air stream from the caustic solution,
- contacting the first purified air stream with water to dissolve residual caustic solution and/or salts in the water,
- separating a second purified air stream from the water containing the residual caustic solution and/or salts, and
- using the second purified air stream in the oxidation of cumene.

2. The process of claim 1, wherein the salts are inorganic salts.

3. The process of claim 1 or claim 2, wherein the water is fresh water.

4. The process of any one of claims 1 to 3, wherein the second purified air stream is used in the dry oxidation of cumene.

5. The process of any one of claims 1 to 4, wherein the first purified air stream is separated from the caustic solution by passing the air through at least two column trays.

6. The process of any one of claims 1 to 5, wherein the air feed is cooled down to a temperature of 20-80°C.

7. The process of any one of claims 1 to 5, wherein the air feed is cooled down to a temperature of 30-60°C.

8. The process of any one of the preceding claims, wherein the aqueous caustic solution is an aqueous sodium hydroxide solution.

9. The process of claim 8, wherein the aqueous sodium hydroxide has a concentration of about 0.1-5%.

10. The process of any preceding claim, wherein the water is essentially free from acidic and alkaline components.

## Patentansprüche

1. Ein Verfahren zur Oxidation von Cumol zu Cumolhydroperoxid, das Verfahren umfasst:
- Bereitstellung einer Luftzufuhr, welche Luft und eine oder mehrere säurehaltige Bestandteile umfasst,
- Kontaktieren der Luftzufuhr mit einem wässrigen kaustischen Lösung, welche zumindest eine alkalische Mischung zur Neutralisierung von zumindest einer der säurehaltigen Bestandteile der Luftzufuhr enthält, wobei Salze gebildet werden,
- Trennen eines ersten gereinigten Luftstroms von der kaustischen Lösung
- Kontaktieren des ersten gereinigten Luftstroms mit Wasser zum Lösen restlicher kaustischer Lösung und/oder Salzen in dem Wasser
- Trennen eines zweiten gereinigten Luftstroms von dem Wasser, das die restliche kaustische Lösung und/oder Salz enthält, und
- Verwendung des zweiten gereinigten Luftstroms zur Oxidation von Cumol.

2. Das Verfahren nach Anspruch 1, wobei die Salze anorganische Salze sind.

3. Das Verfahren nach Anspruch 1 oder Anspruch 2, wobei das Wasser Süßwasser ist.

4. Das Verfahren nach einem der Ansprüche 1 bis 3, wobei der zweite gereinigte Luftstrom in der Trockenoxidation von Cumol verwendet wird.

5. Das Verfahren nach einem der Ansprüche 1 bis 4, wobei der erste gereinigte Luftstrom von der kaustischen Lösung getrennt wird, indem die Luft durch zumindest zwei Rektifikationssäulen hindurch geleitet wird.

6. Das Verfahren nach einem der Ansprüche 1 bis 5, wobei der Luftstrom auf eine Temperatur von 20 bis 80°C herunter gekühlt wird.

7. Das Verfahren nach einem der Ansprüche 1 bis 5, wobei der Luftstrom auf eine Temperatur von 30 bis 60°C herunter gekühlt wird.

8. Das Verfahren nach einem der vorangehenden Ansprüche, wobei die wässrige kaustische Lösung eine wässrige NatriumhydroxidLösung ist.

9. Das Verfahren nach Anspruch 8, wobei die wässrige Natriumhydroxid eine Konzentration von etwas 0,1 bis 5 % aufweist.

10. Das Verfahren nach einem der vorangehenden Ansprüche, wobei das Wasser im Wesentlichen frei von säurehaltigen und alkalischen Bestandteilen ist.

## Revendications

1. Procédé d'oxydation de cumène en hydroperoxyde de cumène, ledit procédé comprenant les étapes consistant à :
- fournir une source d'air qui comprend de l'air et un ou plus de composants acides,
- mettre en contact la source d'air avec une solution caustique aqueuse qui contient au moins un composé alcalin pour neutraliser au moins l'un desdits composés acides dans la source d'air, si bien que des sels se forment,
- séparer un premier courant d'air purifié de la solution caustique,
- mettre en contact le premier courant d'air purifié avec de l'eau pour dissoudre une solution caustique résiduelle et/ou des sels dans l'eau,
- séparer un second courant d'air purifié de l'eau contenant la solution caustique résiduelle et/ou les sels, et
- utiliser le second courant d'air purifié dans l'oxydation du cumène.

2. Procédé selon la revendication 1, dans lequel les sels sont des sels inorganiques.

3. Procédé selon la revendication 1 ou la revendication 2, dans lequel l'eau est de l'eau fraîche.

4. Procédé selon l'une quelconque des revendications 1 à 3, dans lequel le second courant d'air purifié est utilisé dans l'oxydation à sec du cumène.

5. Procédé selon l'une quelconque des revendications 1 à 4, dans lequel le premier courant d'air purifié est séparé de la solution caustique en faisant passer de l'air à travers au moins deux plateaux de colonne.

6. Procédé selon l'une quelconque des revendications 1 à 5, dans lequel la source d'air est refroidie à une température de 20 à 80 °C.

7. Procédé selon l'une quelconque des revendications 1 à 5, dans lequel la source d'air est refroidie à une température de 30 à 60 °C.

8. Procédé selon l'une quelconque des revendications précédentes, dans lequel la solution caustique aqueuse est une solution d'hydroxyde de sodium aqueuse.

9. Procédé selon la revendication 8, dans lequel l'hydroxyde de sodium aqueux a une concentration d'environ 1 à 5 %.

10. Procédé selon l'une quelconque des revendications précédentes, dans lequel l'eau est essentiellement exempte de composants acides et alcalins.
